# EUROPÄISCHE PATENTANMELDUNG

(11) **EP 0 808 631 A1**
(43) Veröffentlichungstag der Anmeldung: **26.11.1997**
(21) Anmeldenummer: 96108360.7
(22) Anmeldetag: 24.05.1996
(51) Int. Cl.: A61L 2/26, A61L 2/24

(54) **Messeinrichtung zum Überwachen von Sterilisationsbedingungen**

(71) Anmelder: INTERMEDICAL S.A.H., 1118 Luxembourg (LU)
(72) Erfinder: Hücker, Gerhard, Dr., 5612 Mondorf-les-Bains (LU)
(74) Vertreter: Grünecker, Kinkeldey, Stockmair & Schwanhäusser Anwaltssozietät

(57) **Zusammenfassung**

Es wird eine Meßeinrichtung (1, 30) zum Überwachen von Sterilisationsbedinungen in einer Sterilisationskammer beschrieben. Die Meßeinrichtung enthält ein außerhalb der Sterilisationskammer verbleibendes Datenaufnahmegerät (3) und ein vom Datenaufnahmegerät (3) getrenntes, in die Sterilisationskammer einsetzbares Meßgerät (2, 31). Das Meßgerät (2, 31) hat ein Gehäuse (4), in dessen Innerem wenigstens eine über einen Zugang (17, 32) mit der Sterilisationskammer in Verbindung stehende Meßeinheit (11, 12, 13) für wenigstens einen Parameter der Sterilisationsbedingungen, eine Stromversorgungseinrichtung (8), eine Steuergerät (9) und ein Datenspeicher (10) untergebracht sind. Das Gehäuse (4) ist mit einem lösbar mit dem Datenaufnahmegerät (3) koppelbaren Datenausgang (14a) versehen. Um diese Meßeinrichtung so zu verbessern, daß eine sichere Dokumentation der Sterilisationsbedingungen möglich ist, wird vorgeschlagen, daß die Meßeinheit einen Sensor (11) zum Messen der relativen Feuchtigkeit aufweist.

## Beschreibung

Die Erfindung bezieht sich auf eine Meßeinrichtung zum Überwachen von Sterilisationsbedingungen in einer Sterilisationskammer der im Oberbegriff des Anspruchs 1 erläuterten Art.

Derartige Meßeinrichtungen sind beispielsweise aus dem DE-U-93 19 369 oder der US-A-5 491 092 bekannt. Die bekannten Meßeinrichtungen bestehen aus zwei Teilen, wobei das eine Teil, das Datenaufnahmegerät, dauernd außerhalb der Sterilisationskammer angeordnet ist und nur das zweite Teil, das eigentliche Meßgerät, den Sterilisationsbedingungen in der Sterilisationskammer ausgesetzt wird. Das Meßgerät enthält eine Reihe von Sensoren, beispielsweise für die Messung der Temperatur, des Drucks und des Wassergehalts des Dampfes am Ende einer als "heat sink" gekennzeichneten Vorrichtung, und wird entweder allein, zum Überprüfen der Funktionsfähigkeit des Sterilisationsgerätes, oder zusammen mit den zu sterilisierenden Gegenständen zur Überprüfung des Sterilisationsprozeßablaufes in die Sterilisationskammer eingelegt. Das Meßgerät bleibt solange in der Sterilisationskammer, bis der Sterilisationsvorgang abgeschlossen ist. Das Meßgerät mißt in vorbestimmten Zeitintervallen Druck, Temperatur und den Wassergehalt des Dampfes und überträgt die Daten über eine Antenne auf das Datenaufnahmegerät bzw. speichert die Daten für eine spätere Übertragung, nachdem das Meßgerät aus der Sterilisationskammer entnommen wurde. Auf diese Weise soll sofort nach Beendigung des Sterilisationsvorganges bzw., wenn die Daten vom Datenaufnahmegerät ausgedruckt werden, auch noch wesentliche später dokumentierbar sein, daß das in der Sterilisationskammer behandelte Gut korrekt und vorschriftsgemäß sterilisiert wurde.

Zur Dokumentation der korrekten Sterilisationsbedingungen stützt man sich bei der bekannten Meßeinrichtung primär auf Druck- und Temperaturmessung, während durch den Feuchtesensor lediglich die Anwesenheit von Wasser im Dampf am Ende einer "heat sink" festgestellt wird. Es hat sich jedoch herausgestellt, daß die bekannte Messung des Wassergehaltes am Ende der "heat sink" allenfalls als flankierende Maßnahme eingesetzt werden kann, da einerseits bei Fehlen von Wasser nicht auf nicht auf die Nicht-Einhaltung der korrekten Sterilisationsbedingungen geschlossen werden kann und andererseits das Auftreten von Wasser nicht auf das Vorhandensein von Sattdampf schließen läßt. Andererseits ist jedoch auch mit einer Temperatur- und Druckmessung allein nicht unmißverständlich sicherzustellen, daß jede Stelle des Sterilisationsgutes den Sterilisationsbedingungen ausgesetzt wurde. Die Messung des Wasseranteils im Dampf ist so ungenau, daß auch aus der Kombination der gemessenen Werte kein Rückschluß auf die Einhaltung der Sterilisationsbedingungen möglich ist.

Der Erfindung liegt somit die Aufgabe zugrunde, eine Meßeinrichtung bereitzustellen, mit der das Vorhandensein von vorbestimmten Sterilisationsbedingungen zuverlässig, d.h. mit der erforderlichen Meßgenauigkeit, feststellbar ist.

Die Aufgabe wird durch die im Anspruch 1 angegebenen Merkmale gelöst.

Durch das zusätzliche Messen der relativen Feuchtigkeit der Luft, wobei als relative Feuchtigkeit das Verhältnis der tatsächlich vorhandenen Wassermasse zu derjenigen Wassermasse definiert ist, die bei derselben Temperatur die Luft sättigen würde, wird auf einfache Weise erreicht, daß ein tatsächlich für die Sterilisation relevanter Parameter gemessen wird. Durch die Messung der relativen Feuchtigkeit der Luft wird beispielsweise derjenige Fehler eliminiert, der auftreten würde, wenn durch eine Druck- und Temperaturmessung Sattdampfbedingungen festgestellt würden, tatsächlich jedoch wegen der Anwesenheit kondensierbarer Gase keine Sattdampfbedingungen vorhanden sind. Dieser Fehler ist durch die Messung des Wasseranteils des Dampfes am Ende einer "heat sink" nicht erkennbar.

Durch die Ausgestaltung nach Anspruch 2 werden weitere eventuelle Fehler eliminiert, da hier nur die tatsächlichen Meßfühler den Sterilisationsbedingungen direkt ausgesetzt sind, während alle anderen Bestandteile in einem gegebenenfalls isolierten, abgeschlossenen Gehäuse untergebracht sind.

Die Ausgestaltung nach Anspruch 3 stellt sicher, daß sich an allen Meßfühlern, und insbesondere auch am Feuchtesensor kein Kondensat niederschlagen kann. Durch den Dampfverteiler wird sichergestellt, daß der Sterilisationsdampf dem Meßraum allseitig zugeführt wird, so daß Temperaturschwankungen, die zum Auskondensieren führen könnten, zumindest außerhalb der Anfangsphase des Sterilisationsprozesses vermieden werden.

Anspruch 4 beschreibt eine besonders bevorzugte konstruktive Ausgestaltung eines derartigen Dampfverteilers.

Auch die erfindungsgemäße Meßeinrichtung ist gemäß Anspruch 5 mit einer Einrichtung zum erschwerten Eintritt der Sterilisationsbedingungen versehen, die beispielsweise die Evakuierung englumiger, langer Instrumente und Kanülen verzögern.

Diese Einrichtung kann gemäß Anspruch 6 einen Schlauch enthalten, der eine Evakuierung der zu sterilisierenden Instrumente und Geräte verzögert, wenn mit Druckwechsel gearbeitet wird.

Um zu verhindern, daß das Innere der Meßkammer als Pumpe wirkt, sollten die Volumina der Meßkammer und des Schlauches gemäß Anspruch 7 aufeinander abgestimmt werden.

Die Einrichtung zum Verzögern der Übertragung der Sterilisationsbedingungen kann jedoch auch, alternativ und zusätzlich zum Schlauch, das in Anspruch 8 beschriebene Paket enthalten, das das Eindringen von Dampf verzögert, so wie es z.B. bei einem kompakten Wäschepaket der Fall ist.

Anspruch 9 beschreibt eine bevorzugte Anordnung des Paketes, wenn dieses zusammen mit dem Schlauch verwendet wird.

Anspruch 10 beschreibt eine besonders bevorzugte Möglichkeit, die Daten zwischen dem Meßgerät und dem Datenaufnahmegerät zu übertragen.

Durch die Ausgestaltung nach Anspruch 11 kann beispielweise die Frequenz der Messungen oder die Art der Messungen, die das Meßgerät durchzuführen hat, vorab bestimmt und von Fall zu Fall geändert werden.

Anspruch 12 beschreibt ein Ausführungsbeispiel der erfindungsgemäßen Meßeinrichtung, die speziell zur Verwendung beim Gravitationsverfahren oder Strömungsverfahren ausgebildet ist, bei dem kein Druckwechsel stattfindet, sondern der Dampf einfach in die Sterilisationskammer eingeleitet wird und dort die Luft verdrängt bzw. einmalig vorab evakuiert wird.

Ausführungsbeispiele der Erfindung werden nachfolgend anhand der Zeichnungen näher erläutert. Es zeigen:
- Fig. 1: eine schematische Darstellung eines ersten Ausführungsbeispiels einer erfindungsgemäßen Meßeinrichtung, und
- Fig. 2: eine schematische Darstellung eines zweiten Ausführungsbeispiels der erfindungsgemäßen Meßeinrichtung.

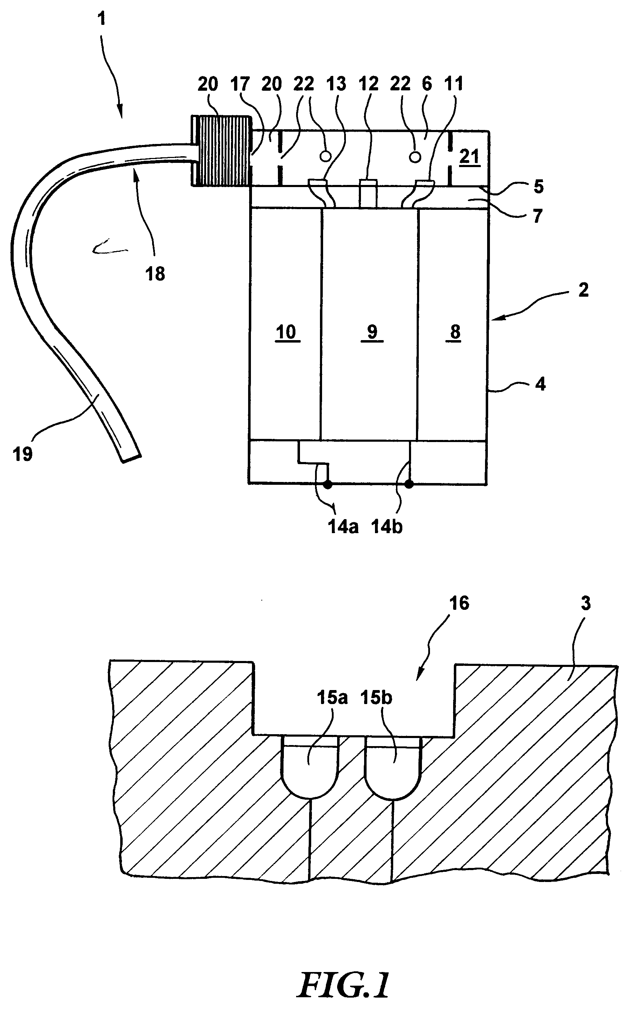

Fig. 1 zeigt eine erste Meßeinrichtung 1, die sich insbesondere zur Verwendung beim Sterilisieren nach dem sogenannten Vorvakuumverfahren eignet. Die erfindungsgemäße Meßeinrichtung 1 enthält eine Meßgerät 2 und ein Datenaufnahmegerät 3. Das Meßgerät 2 enthält ein äußeres Gehäuse 4 aus Edelstahl, Kunststoff oder einem anderen geeigneten, druck- und temperaturbeständigen sowie inerten Material. Das Gehäuse 4 ist durch eine Trennwand 5 in einen Meßraum 6 und eine in sich abgeschlossene Kammer 7 unterteilt. Die Kammer 7, die gegebenenfalls isoliert sein kann, enthält im dargestellten Ausführungsbeispiel ein Aggregat zur Energieversorgung, beispielsweise einen Akku 8, ein Steuergerät 9 und einen Speicher 10. Der Akku 8 kann beispielsweise über nicht gezeichnete, sich durch das Gehäuse 4 erstreckende Ladekontakte aufgeladen werden. Die Energieversorgung kann jedoch auch über eine Batterie erfolgen, die in ein im Gehäuse 4 vorgesehenes Batteriefach auswechselbar eingelegt werden kann.

Das Steuergerät 9 ist mit einer Reihe von Meßeinheiten in Form von Meßfühlern verdrahtet, die sich durch die Zwischenwand 5 erstrecken und mit dem Meßraum 6 in Verbindung stehen. Im dargestellten Ausführungsbeispiel ist ein Luft-Feuchtesensor 11, ein Drucksensor 12 und ein Temperatursensor 13 vorgesehen. Alle Sensoren sind handelsüblich. Der Luft-Feuchtesensor 11 ist so ausgebildet und wird derart betrieben, daß mit ihm die relative Feuchtigkeit, d.h. das Verhältnis der tatsächlich vorhandenen Feuchtigkeit zu derjenigen Feuchtigkeit, die bei derselben Temperatur die Luft sättigen würde, meßbar ist. Die gemessenen Daten werden dem Steuergerät 9 zugeführt und dort verarbeitet, wobei das Steuergerät 9 unter anderem einen Zähler zum Abzählen der vorbestimmten Anzahl der Meßpunkte, einen Zeitgeber, zum Vorgeben vorbestimmter Zeitintervalle und/oder einen Echtzeitgeber zum Dokumentieren der Echtzeit enthalten kann. Die ermittelten Daten werden im Speicher 10 gespeichert.

Der Speicher 10 ist mit einem optischen Datenausgang 14a und das Steuergerät 9 mit einem optischen Dateneingang 14b verbunden, die der Übertragung von Daten an das und von dem Datenaufnahmegerät 3 dienen. Zu diesem Zweck hat das Datenaufnahmegerät 3 einen Dateneingang 15a und einen Datenausgang 15b, die zweckmäßigerweise in einer Vertiefung 16 angeordnet sind, in die das Gehäuse 4 nur in einer vorbestimmten Position eingeschoben werden kann, so daß eine korrekte Datenübermittlung sichergestellt wird. Optische Datenübertragungen sind im Stand der Technik bekannt und bedürfen keiner weiteren Erläuterung. Das Datenaufnahmegerät 3 kann weiterhin mit den erforderlichen Anzeigeinrichtungen zum sofortigen Anzeigen der vom Meßgerät 2 übernommenen Daten, mit einem Drucker zum Ausdrucken dieser Daten und mit entsprechenden Bedienungselementen versehen sein, die es gestatten, das Steuergerät 9 zu programieren, d.h. beispielsweise das Zeitintervall der Messungen, die Art der Messungen oder dgl. vorzugeben bzw. diese Vorgaben zu verändern und auf die speziell zu sterilisierenden Gegenstände abzustimmen. Auch die obenbeschriebenen Elemente des Datenaufnahmegerätes 3 sind im Stand der Technik bekannt und können vom Fachmann für den vorgegebenen Zweck ausgewählt und eingesetzt werden.

Im Ausführungsbeispiel nach Fig. 1 steht der Meßraum 6 mit der Umgebung über eine sich durch das Gehäuse 4 erstreckende Zugangsöffnung 17 in Verbindung. Außen am Gehäuse 4 und die Zugangsöffnung 17 überdeckend ist eine Einrichtung 18 zur verzögerten Übertragung der Sterilisationsbedingungen in den Meßraum 6 lösbar und auswechselbar befestigt. Die Einrichtung 18 enthält einen flexiblen, langen und dünnen Schlauch 19, wie er beispielsweise auch für die Routineüberwachung der Sterilisation mittels Bioindikatoren verwendet wird, und ein Paket 20 aus einem Papier-, Baumwoll-, Faser- oder Partikelmaterial. Der Schlauch 19 kann in Länge und Durchmesser an das zu sterilisierende Gut bzw. an die Leistungsfähigkeit des Sterilisators angepaßt werden und weist eine Länge auf, die etwa in der Größenordnung zwischen 0,5 und 2,5 m liegt. Der Innendurchmesser des Schlauches kann im Bereich zwischen einem und wenigen Millimetern liegen, beispielsweise 2 mm sein.

Das Paket 20 besteht im dargestellten Ausführungsbeispiel aus einer Mehrzahl von Lagen eines Fasermaterials, wie beispielsweise Papier, Vliesstoff, Baumwolle oder dgl. Das Paket 20 ist zwischen dem Schlauch 19 und der Zugangsöffnung 17 so angeordnet, daß die durch den Schlauch 19 eintretenden Gase bzw. der Dampf zunächst durch das Paket 20 und erst dann in die Zugangsöffnung 17 gelangt. Das Paket kann auch ohne vorliegenden Schlauch direkt von der Zugangsöffnung angewendet werden.

Die Zugangsöffnung 17 mündet im Inneren des Meßraumes 6 in einen Dampfverteiler 20. Der Dampfverteiler 20 enthält eine sich um den gesamten Umfang der Meßkammer 6 erstreckende Verteilungsleitung 21, die über eine Mehrzahl gleichmäßig bzw. in einer speziellen Anordnung um den Umfang des Meßraumes 6 verteilte Dampföffnungen 22 mit dem Meßraum 6 in Verbindung steht. Dadurch gelangt der Dampf über die Zugangsöffnung 17 zunächst in die Verteilleitung 21, wird gleichmäßig um den Umfang des Meßraumes 6 und somit um den Umfang der Meßfühler 11 bis 13 verteilt und dann durch die Dampföffnungen 22 allseitig so eingeblasen, so daß eine unerwünschte Kondensatbildung so weit wie möglich vermieden wird. Außerdem wird dadurch sichergestellt, daß die Verzögerung der Übertragung der Sterilisationsbedingungen allein durch die Einrichtung 18, d.h. die Kombination aus Schlauch 19 und Paket 20 erfolgt, oder einer von beiden, während am eigentlichen Meßort in der Meßkammer 6 sowohl eine gleichmäßige Dampfverteilung ohne wesentliche Kondensatbildung als auch eine zuverlässige Druck- und Temperaturübertragung stattfindet. Das freie, d.h. durch Dampf füllbare Volumen des Meßraumes 6 sollte klein gehalten werden, um sicherzustellen, daß der Meßraum 6 nicht als Pumpe wirkt. Es wurde festgestellt, daß zu diesem Zweck das Volumen des freien Raums innerhalb des Meßraumes 6 kleiner oder gleich sein soll dem Volumen eines Schlauchstückes von einer derartigen Länge, die der Länge des Meßraumes 6 senkrecht zur Zugangsöffnung 17 bzw. dem Luftinhalt des Paketes entspricht.

Die erfindungsgemäße Meßeinrichtung wird verwendet, indem das Meßgerät 2, gegebenenfalls nach dem Programmieren und mit dem dem entsprechenden Sterilisiergut angepaßten Schlauch oder Paket bzw. der angepaßten Kombination aus Schlauch und Paket, in die nicht gezeichnete Sterilisationskammer eines nicht gezeichneten Sterilisationsgerätes eingelegt wird. Während des Sterilisationsvorganges werden Druck, Temperatur und relative Luftfeuchtigkeit in vorbestimmten Zeitintervallen gemessen und gespeichert, gegebenenfalls zusammen mit Informationen über die Echtzeit bzw. den Zeitablauf. Nach Beendigung des Sterilisationsvorganges wird das Meßgerät 2 der Sterilisationskammer entnommen und in die Ausnehmung 16 des Datenaufnahmegerätes 3 eingesetzt. Die gespeicherten Daten werden übertragen und entweder angezeigt oder ausgedruckt. Den auf das Datenaufnahmegerät 3 übertragenen Daten können weiterhin Informationen über das Sterilisiergut zugeordnet werden, beispielsweise durch Einlesen eines am Sterilisiergut angeordneten und das Sterilisiergut identifizierenden Barcodes oder dgl. Damit ist eine sichere und unverwechselbare Dokumentation der Sterilisationsbedingungen möglich, die es auch noch nach längerer Zeit gestattet, festzustellen, ob das spezielle Sterilsiergut den Vorschriften entsprechend sterilisiert wurde.

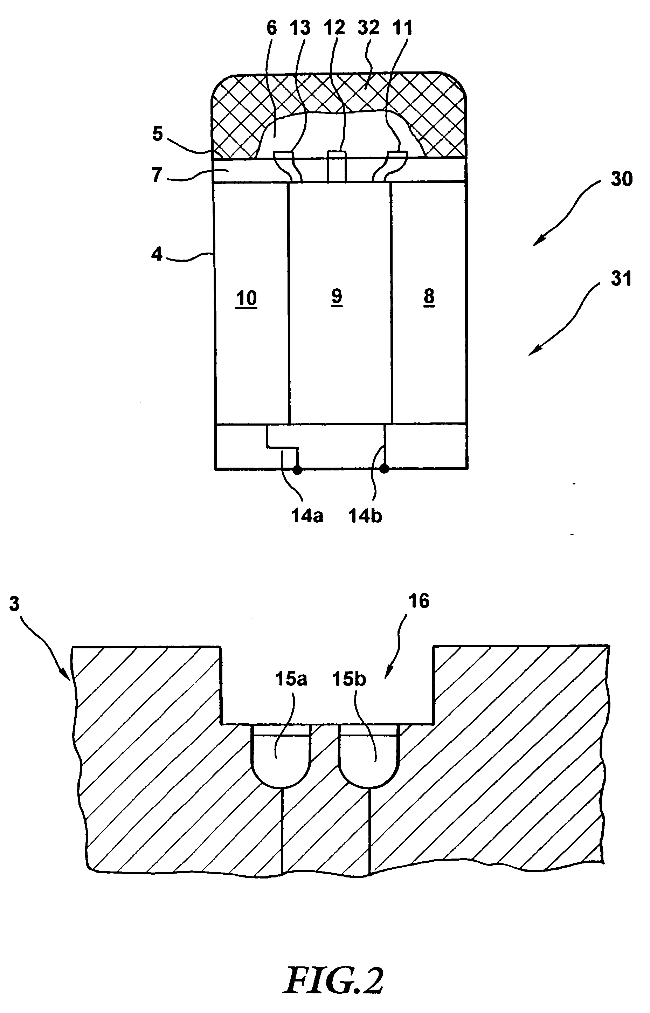

Fig. 2 zeigt ein Ausführungsbeispiel einer Meßeinrichtung 30, die speziell zur Verwendung in Sterilisationsgeräten ausgebildet wurde, die nach dem Gravitationsverfahren, d.h. ohne Druckwechsel, arbeiten. Die Meßeinrichtung 30 entspricht der Meßeinrichtung 1 in dem durch gleiche Bezugszeichen gekennzeichneten Teilen, enthält jedoch ein abgewandeltes Meßgerät 31. Das Meßgerät 31 enthält die gleiche, durch eine Trennwand 5 im Gehäuse 4 abgetrennte Kammer 7 mit den gleichen Bestandteilen wie im vorangegangenen Ausführungsbeispiel, unterscheidet sich jedoch durch die Ausgestaltung oberhalb der Trennwand 5. Der Meßraum 6 des Meßgerätes 31 ist nicht abgeschlossen, sondern enthält eine sieb- oder korbartig durchbrochene Abdeckung 32 über den Meßeinheiten 11 bis 13. Die durchbrochene Abdeckung 32 ist in ihrer Durchlässigkeit so ausgebildet, daß sie ebenfalls als Dampfverteilter wirkt, und beispielsweise konzentrierte Dampfstrahlen verteilt und somit ebenfalls Kondensation verhindert. Auch auf diese Weise kann der Feuchtesensor 11 zur Bestimmung der relativen Feuchtigkeit korrekt arbeiten. Die Durchlässigkeit wird eingestellt über die Querschnittsgröße der Öffnungen und die Öffnungsdichte pro Flächeneinheit. Diese sollten so groß sein, daß ein guter Dampfaustausch gewährleistet ist, sie sollten jedoch klein genug sein, um zu verhindern, daß Wassertropfen hindurchtreten.

Die Erfindung ist nicht auf die beschriebenen und gezeichneten Ausführungsbeispiele beschränkt. So kann beispielsweise die Einrichtung zur Verzögerung der Übertragung der Sterilisationsbedingungen nur den Schlauch, nur das Paket oder eine andere im Stand der Technik bekannte Einrichtung enthalten. Auch der Dampfverteiler gemäß Fig. 1 kann anstelle der Dampfeintrittsöffnungen eine siebartige Wand aufweisen. Auch die Datenübertragung kann durch andere, bekannte Datenübertragungseinrichtungen erfolgen.

## Patentansprüche

1. Meßeinrichtung (1, 30) zum Überwachen von Sterilisationsbedingungen in einer Sterilisationskammer, mit einem außerhalb der Sterilisationskammer verbleibenden Datenaufnahmegerät (3) und einem vom Datenaufnahmegerät (3) getrennt in die Sterilisationskammer einsetzbaren Meßgerät (2, 31), wobei das Meßgerät (2, 31) ein Gehäuse (4) aufweist, in dessen Innerem wenigstens eine über einen Zugang (17, 32) mit der Sterilisationskammer in Verbindung stehenden Meßeinheit (11, 12, 13) für wenigstens einen Parameter der Sterilisationsbedingungen, eine Stromversorgungseinrichtung (8), ein Steuergerät (9) und ein Datenspeicher (10) untergebracht sind, und das Gehäuse (4) mit einem lösbar mit dem Datenaufnahmegerät (3) koppelbaren Datenausgang (14a) versehen ist, **dadurch gekennzeichnet**, daß die Meßeinheit einen Sensor (11) zum Messen der relativen Feuchtigkeit aufweist.

2. Meßeinrichtung nach Anspruch 1, **dadurch gekennzeichnet**, daß im Gehäuse (4) des Meßgerätes (2, 31) ein über die Zugangsöffnung (17, 32) zur Sterilisationskammer offener Meßraum (6), in dem die Meßeinheiten (11, 12, 13) untergebracht sind, und eine gegenüber der Sterilisationskammer abgeschlossene Kammer (7) vorgesehen ist, in der die Stromversorgungseinrichtung (8), das Steuergerät (9) und der Datenspeicher (10) untergebracht sind.

3. Meßeinrichtung nach Anspruch 1 oder 2, **dadurch gekennzeichnet**, daß zwischen der Sterilisationskammer und dem Meßraum (6) ein Dampfverteiler (21, 32) angeordnet ist.

4. Meßeinrichtung nach Anspruch 3, **dadurch gekennzeichnet**, daß der Dampfverteiler eine mit einer Mehrzahl von Austrittsöffnungen (22) versehene, sich um den Umfang des Meßraums erstreckenden, ringförmigen Verbindungsleitung (21) aufweist.

5. Meßeinrichtung nach einem der Ansprüche 1 bis 4, **dadurch gekennzeichnet**, daß die Zugangsöffnung (17) zum Meßraum (6) des Meßgerätes (2) mit einer eine erschwerende Übertragung der Sterilisationsbedinungen aus der Sterilisationskammer zur Meßeinheit (11, 12, 13) bewirkenden Einrichtung (18) versehen ist.

6. Meßeinrichtung nach Anspruch 5, **dadurch gekennzeichnet**, daß die Einrichtung (18) einen Schlauch (19) zur Verzögerung der Evakuierung aus dem Meßraum (6) enthält.

7. Meßeinrichtung nach Anspruch 6, **dadurch gekennzeichnet**, daß das Volumen des freien Raums innerhalb des Meßraumes (6) gleich oder kleiner dem Volumen eines der Länge des Meßraums (6) entsprechend langen Stücks des Schlauches (19) ist.

8. Meßeinrichtung nach einem der Ansprüche 5 bis 7, **dadurch gekennzeichnet**, daß die Einrichtung (18) ein Paket (20) aus einem gepackten Faser- oder Partikelmaterial zur Verzögerung der Dampfeindringung in den Meßraum (6) enthält.

9. Meßeinrichtung nach Anspruch 8, **dadurch gekennzeichnet**, daß das Paket (20) zwischen dem Schlauch (19) und der Zugangsöffnung (17) zum Meßraum (6) angeordnet ist.

10. Meßeinrichtung nach einem der Ansprüche 1 bis 9, **dadurch gekennzeichnet**, daß der Datenausgang (14a) für eine optische Datenübertragung ausgebildet ist.

11. Meßeinrichtung nach einem der Ansprüche 1 bis 10, **dadurch gekennzeichnet**, daß das Meßgerät (2, 31) programmierbar ist.

12. Meßeinrichtung nach einem der Ansprüche 1 bis 3, **dadurch gekennzeichnet**, daß wenigstens ein Teil der Wandung des Meßraums (6) zur Sterilisationskammer aus einem durchbrochenen Material (32) besteht.
